# EUROPÄISCHE PATENTSCHRIFT

(11) **EP 0 959 834 B1**
(45) Veröffentlichungstag und Bekanntmachung des Hinweises auf die Patenterteilung: **13.02.2002**
(21) Anmeldenummer: 96942340.9
(22) Anmeldetag: 04.12.1996
(51) Int. Cl.: A61F 13/02

(54) **VERFAHREN ZUR HERSTELLUNG TRANSDERMALER THERAPEUTISCHER PFLASTER (TTS)**
METHOD FOR PRODUCING TRANSDERMAL PATCHES (TTS)
PROCEDE DE PRODUCTION DE SYSTEMES THERAPEUTIQUES TRANSDERMIQUES

(30) Priorität: 20.12.1995 DE 19547691
(43) Veröffentlichungstag der Anmeldung: 01.12.1999
(73) Patentinhaber: LTS LOHMANN Therapie-Systeme AG, 56626 Andernach (DE)
(72) Erfinder: ASMUSSEN, Bodo, D-56170 Bendorf (DE); HILLE, Thomas, D-56567 Neuwied (DE); SCHUMANN, Klaus, D-56567 Neuwied (DE); STEINBORN, Peter, D-56567 Neuwied (DE)
(74) Vertreter: Flaccus, Rolf-Dieter, Dr.
(86) Internationale Anmeldenummer: EP9605410
(87) Internationale Veröffentlichungsnummer: WO9722315

(56) Entgegenhaltungen:
- WO-A-95/23687
- DE-C- 4 232 279
- GB-A- 2 237 262

## Beschreibung

Die Erfindung betrifft ein Verfahren zur Herstellung transdermaler therapeutischer Pflaster (TTS) nach dem Oberbegriff des Patentanspruches 1.

Durch die DE-PS 32 04 582 ist ein Verfahren zum kontinuierlichen Herstellen und Füllen von Pflasterverpackungen mit einem transdermal zu verabreichenden Wirkstoff bekannt. Dabei wird der Wirkstoff portionsweise in bestimmten Abständen auf eine Trägerfolie aufgebracht, mittels metallischer Deckfolienabschnitte abgedeckt und dann unter Verwendung einer Zwischenlagefolie mit einer elastischen, klebseitig gegen diese gerichteten, einseitig klebefähigen Haftfolie überdeckt und sodann die Wirkstoffportionen mit den sie umgebenden Folienschichten in der gewünschten Größe ausgestanzt. Dieses Verfahren bedingt einen verhältnismäßigen großen technischen Aufwand und ist mit verhältnismäßig großen Mengen überflüssigen Materials behaftet.

Bekannt ist auch ein Verfahren zur kontinuierlichen Herstellung transdermaler therapeutischer Pflaster, bei dem zunächst durch Beschichten einer Zwischenträgerfolie mit einer fließfähigen, wirkstoffhaltigen Zubereitung ein Laminat hergestellt und dieses dann zu Streifen vorgegebener Breite und in weiteren Verfahrensschritten zu wirkstoffhaltigen Abschnitten bestimmter Länge zurechtgeschnitten,diese in vorgegebenen Abständen auf eine sie allseitig überdekkende Schutzfolie aufgebracht und schließlich durch Trennen der Schutzfolie quer zum Bandverlauf zwischen den wirkstoffhaltigen Abschnitten in Einzelpflaster unterteilt werden. Dieses Verfahren löst zwar sehr vorteilhaft die gestellte Aufgabe, Wirkstoffverluste weitgehend zu unterbinden bzw., zu minimieren, sie ist jedoch wegen der vielen Verfahrens schritte verhältnismäßig umständlich.

Ein weiteres Verfahren ist in der DE - OS 41 10 027 beschrieben. Darin wird ein Verfahren zur kontinuierlichen Herstellung transdermaler therapeutischer Pflaster beschrieben, die eine Rückschicht, eine haftklebende Wirkstoffreservoirschicht und eine ablösbare Schutzschicht aufweisen, wobei Wirkstoffverlust während der Konfektionierung minimiert wird. Bei diesem Verfahren wird das haftklebende Wirkstoffreservoir in Form eines Laminates, bestehend aus einer wirkstoffhaltigen Haftklebeschicht und einer Polymerfolie , mittels Spenderkante auf die spätere Schutzschicht übertragen. Nachteilig an diesem Verfahren ist jedoch, daß das Endprodukt in jedem Falle zwei Polymerfolien nach Entfernen der Schutzschicht aufweist, was dem gesamten TTS eine so große Starrheit verleiht, daß nicht in jedem Falle ein zufriedenstellender Tragekomfort gegeben ist.

Ziel der vorliegenden Erfindung ist es daher, vereinzelte wirkstoffhaltige Abschnitte mit hoher Geschwindigkeit, großer Genauigkeit und ohne Wirkstoffverlust von einer ersten Bahn in vorherbestimmten Abständen hintereinander auf eine die Abschnitte bevorzugt allseitig überragende zweite Bahn in technisch einfacher und zuverlässiger Weise zu übertragen, wobei vermieden werden muß, daß der Tragekomfort des Endproduktes durch eine innerhalb des TTS liegenden Polymerfolie beeinträchtigt wird.

Diese Aufgabe wird erfindungsgemäß mit einem Verfahren gemäß Patentanspruch 1 gelöst. Diese Lösung ist umso erstaunlichher, als es bisher unter Fachleuten als feststehende Regel zum technischen Handeln galt, daß Haftkleberfilme nur in Form von Laminaten mit steifen Flächengebilden übertragen werden können.

Es sind in der DE-OS 41 10 027, der DE-OS 15 11 873, der DE-PS 25 55 910, der DE-OS 32 33 546, der DE-PS 36 18 542 und der DE 42 32 279 zwar Transferverfahren beschrieben, in welchen jedoch nie die Möglichkeit aufgezeigt wird, beim Übertragen des selbstklebenden Laminates die Prozeßfolie zu entfernen.

Bei der Herstellung des erfindungsgemäßen Pflasters kann je nach Bedarf, d.h. je nachdem, wofür das Pflaster Verwendung finden soll, eine mehr oder weniger große Anzahl von Schichten vorgesehen werden, wobei für die einzelnen Schichten jeweils geeignete Materialien wie Metalle, vorzugsweise Aluminium, Polymere oder auch textile Flächengebilde in Betracht kommen.
Die einzelnen Schichten können dabei entsprechend ihrem jeweiligen Zweck selbstklebend oder auch klebstoffabweisend, wirkstoffdurchlässig oder auch wirkstoffundurchlässig, flexibel oder nicht flexibel ausgebildet sein.

Dabei können wirkstoffhaltige Abschnitte unterschiedliche Formen wie rechteckig, quadratisch, oval oder kreisförmig aufweisen. Im Hinblick auf die Vermeidung von Wirkstoffverlusten ist jedoch eine rechteckige oder quadratische Form bevorzugt.

Von den zur Herstellung der wirkstoffhaltigen Schicht verwendeten Polymeren und dem Wirkstoff abhängige mögliche Zusätze sind beispielsweise Weichmacher, Klebrigmacher, Stabilisatoren, Trägerstoffe, diffusions- und penetrationsregulierende Zusätze oder Füllstoffe. Die in Frage kommenden physiologisch unbedenklichen Substanzen sind dem Fachmann bekannt. Die Eigenklebrigkeit der wirkstoffhaltigen Schicht soll einen dauernden Kontakt zur Haut sicherstellen.

Eine vor der Anwendung abzulösende Schutzschicht der wirkstoffhaltigen Schicht kann beispielsweise aus den selben Materialien wie die zur Herstellung der Rückschicht verwendeten bestehen. Diese müssen jedoch - beispielsweise durch eine Silikonbehandlung - ablösbar gemacht werden. Andere ablösbare Schutzschichten sind beispielsweise Tetrafluorethylen, behandeltes Papier, Cellophan, Polyvinylchlorid und ähnliche.

Die Prozeß- und Hilfsschichten können aus denselben Materialen hergestellt sein.
Die Haftkleberschichten können beispielsweise aus einer Polymermatrix mit einem Grundpolymer und gegebenenfalls üblichen Zusätzen hergestellt sein. Geeignete Materialien sind z.B. Silikone, Kautschuk, kautschukähnliche synthetische Homo-, Co-, oder Blockpolymere, Polyacrylate und deren Copolymere und außerdem Ester von hydriertem Colophonium. Grundsätzlich kommen alle Polymere in Frage, die bei der Herstellung von Haftklebern eingesetzt werden und physiologisch unbedenklich sind. Besonders bevorzugt sind solche, die als Blockcopolymere auf Basis von Styrol und 1,3-Dien, Polyisobutylen oder Polymeren und Copolymeren aus Acrylat und/oder Methacrylat bestehen. Von den Blockcopolymeren auf Basis von Styrol und 1,3-Dien finden bevorzugt lineare Styrol-Isopren-Styrol-Blockcopolymere Verwendung.

Als Wirkstoffe werden Substanzen verwendet, die ohne oder mit Resorptionsvermittler auf der Haut appliziert werden und eine lokale oder systemische Wirkung hervorrufen.

Stoffe mit lokaler Wirkung sind z.B. Antitranspirantia, Fungizide, Bakterizide und Bakteriostatika.

Stoffe mit systemischer Wirkung sind beispielsweise Antibiotika, Hormone, Antipyretika, Antidiabetika, Coronardilatatoren, herzwirksame Glykoside, Spasmolytika, Antihyportonika, Psychopharmaka, Migränemittel, Cortikoide, Analgetika, Kontrazeptiva, Antirheumatika, Cholinergika oder Anticholinergika, Symphatikomimetika oder Symphatikolytika, Vasodilatatoren, Antikoagulantien oder Antiarrhythmika. Selbstverständlich kommen auch andere Wirkstoffe in Frage.

Die Erfindung ist in der Zeichnung in Ausführungsbeispielen gezeigt und wird anhand dieser im folgenden beispielhaft erläutert.

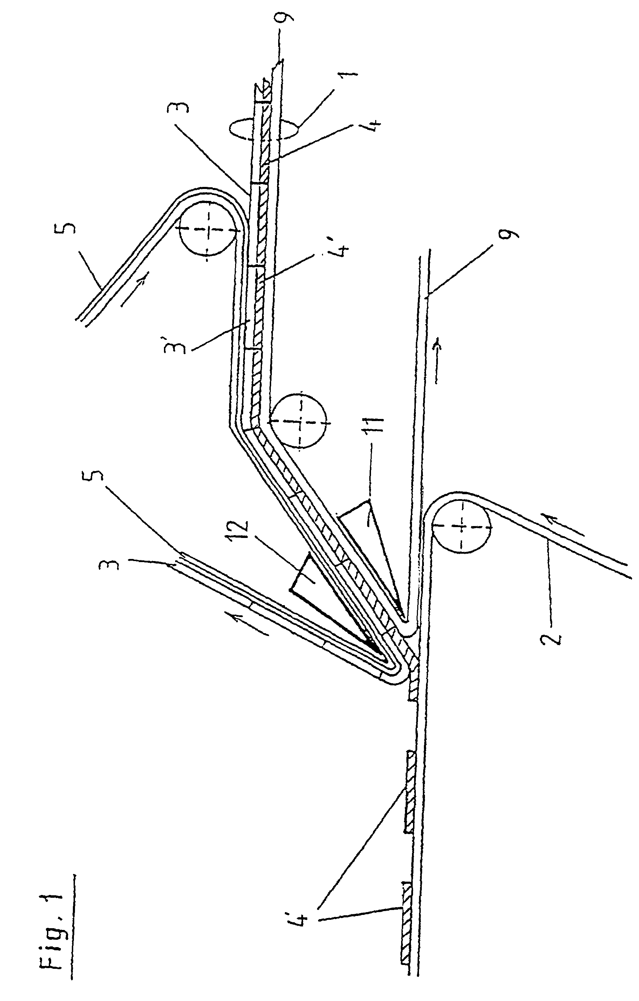

In Figur 1 bezeichnet (1) das erste bahnförmige Laminat bzw. die erste Bahn, wobei diese von oben nach unten aus einer mindestens einseitig silikonisierten Hilfsschicht aus Folie (3), einer wirkstoffhaltigen haftklebenden Schicht (4) und einer Trägerschicht (9) (mindestens einseitig silikonisiert) besteht.

Mit einer Schneidvorrichtung wird die Folie (3) und die wirkstoffhaltige Schicht (4) senkrecht zur Bahnrichtung mit einem geraden Schnitt durchtrennt, so daß beispielsweise quadratische Abschnitte (3',4') entstehen. Die silikonisierte Trägerschicht (9) wird dabei nicht durchtrennt. Mit Hilfe eines Zangenvorzugs, anstelle dessen auch ein Rollenvorzug oder dergleichen vorgesehen sein kann, wird die erste Bahn (1) in der Bewegungsphase von rechts nach links transportiert und dann mit Hilfe eines Niederhalters festgehalten. Unmittelbar nach dem Durchtrennen der Schichten (3) und (4) wird das Laminat (5), bestehend aus einer haftklebend ausgerüsteten Prozeßfolie dem bahnförmigen Laminat (1) auf der Seite der Schicht (3) zukaschiert. An der ersten Spenderkante (11) der Transfervorrichtung werden die wirkstoffhaltigen haftklebenden Abschnitte (4') der Schicht (4) von der Trägerfolie (9) gelöst.

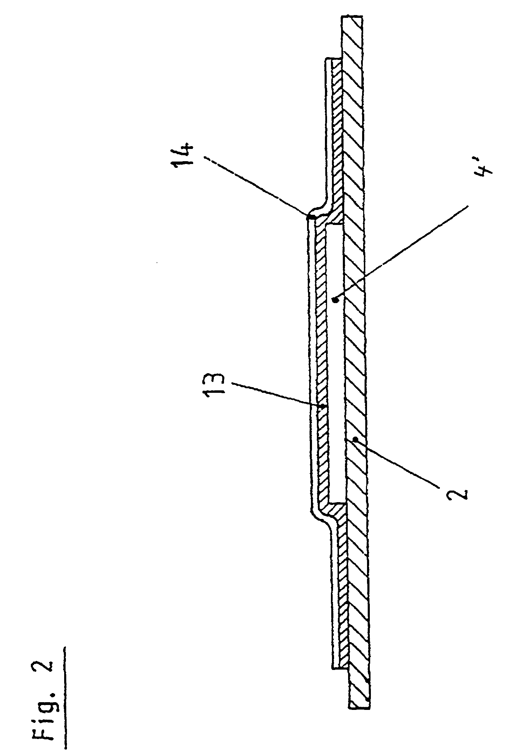

Über eine zweite Spenderkante (12) werden nun die Abschnitte (3') der Schicht (3) von den wirkstoffhaltigen haftklebenden Abschnitten (4') abgezogen. Danach haften die Abschnitte (4') auf der zweiten Bahn (2).
Die Bewegungsrichtungen aller Bahnen sind durch Pfeile angedeutet.
Über die gesamte Fläche der zweiten Bahn (2) wird ein Laminat, bestehend aus einer wirkstoffreien Haftkleberschicht (13) und einer wirkstoffundurchlässigen Folie (14) (Rückschicht) kaschiert (Fig.2). Man stanzt die Einzelpflaster aus, indem man die Schichten (13) und (14),aber nicht (2) durchtrennt.
Der dabei zwischen den Abschnitten (4') anfallende gitterartige Verschnitt der Schichten (13) und (14) wird abgezogen. Durch Querschneiden von (2) erhält man dann die Einzelpflaster (Fig.2).

Die wirkstoffhaltige haftklebende Schicht (4) kann beispielsweise bei einer Stärke von 100 g/m² 57 % Lösungspolyacrylat, 25 % Weichmacher, 10 % Polymethacrylat und 8 % Physostigmin enthalten. Bei einer Bahnbreite von 35 mm besteht die Möglichkeit, quadratische Pflasterformate (4') von 35 x 35 mm zu erhalten. Die zweite Bahn (2) ist dabei ca. 55 mm breit und kann aus beidseitig silikonisierter Polyester-Folie (PET) gebildet sein.

## Patentansprüche

1. Verfahren zur kontinuierlichen Herstellung transdermaler therapeutischer Pflaster, bei dem ein Laminat aus einer mindestens einseitig silikonisierten Hilfsschicht (3), einer wirkstoffhaltigen haftklebenden Schicht (4) und einer Trägerschicht (9) als streifenförmige Bahn (1) vorgelegt und die durch Stanzen der Schichten (3) und (4) quer zur Bahnrichtung erhaltenen wirkstoffhaltigen Abschnitte (4') taktweise auf eine zweite Bahn (2) übertragen werden, wobei der Stanzvorgang in der Ruhephase zwischen den Takten stattfindet und der Übertragungsvorgang auf die zweite Bahn (2) in vorzugsweise gleichen Abständen mittels einer Übergabeeinrichtung (11,12) vorgenommen wird, **dadurch gekennzeichnet, daß** beim Transfer zuerst die Trägerschicht (9) von den wirkstoffhaltigen Abschnitten (4') und danach die Abschnitte (3') der Hilfsschicht unter Zuhilfenahme einer haftklebend ausgerüsteten Prozeßschicht (5) abgezogen werden.

2. Verfahren nach Anspruch 1, **dadurch gekennzeichnet, daß** die streifenförmige Bahn (1) und die zweite Bahn (2) mit unterschiedlicher Bewegungs- und Ruhephase und/oder unterschiedlicher Schrittlänge und/oder unterschiedlicher Geschwindigkeit taktweise vorwärts bewegt werden.

3. Verfahren nach Anspruch 1, **dadurch gekennzeichnet, daß** die Übergabeeinrichtungen bei dem Transfer der wirkstoffhaltigen Abschnitte (4') in Bahnrichtung hin- und herbewegt werden.

## Claims

1. A process for the continuous production of transdermal therapeutic patches, wherein a laminate of an auxiliary layer (3) siliconized at least on one side, an active substance-containing, pressure-sensitive adhesive layer (4), and a supporting layer (9) is prepared as a strip-shaped web (1) first, and the active substance-containing sections (4') obtained by punching said layers (3) and (4) across the web direction are intermittently transferred on a second web (2), the punching procedure taking place in the rest phase between the cycles and the transfer procedure onto the second web (2) being carried out preferably at equal distances by means of a transfer device (11, 12), **characterized in that** the supporting layer (9) is stripped from the active substance-containing sections (4') during the transfer first, and that then the sections (3') of the auxiliary layer are removed with the aid of a process layer (5) which has been rendered pressure-sensitive adhesive.

2. The process according to claim 1 **characterized in that** the strip-shaped web (1) and the second web (2) are intermittently forwarded at differing movement and rest phases, and/or differing step lengths, and/or differing rates.

3. The process according to claim 1 **characterized in that** the transfer devices are moved to-and-fro in web direction during the transfer of the active substance-containing sections (4').

## Revendications

1. Procédé pour la préparation en continu de pansements thérapeutiques transdermiques, dans lequel on dispose au préalable un laminat constitué d'une couche auxiliaire (3) siliconée d'au moins un côté, d'une couche adhésive (4) contenant une ou plusieurs substances actives et d'une couche de support (9) sous forme d'une bande (1) en forme de ruban et dans lequel on transfère en cycle les parties (4') contenant une ou plusieurs substances actives obtenues par découpage des couches (3) et (4) transversalement au sens de la bande sur une deuxième bande (2), le processus de découpage ayant lieu dans la phase de repos entre les cycles et le processus de transfert sur la deuxième bande (2) étant de préférence réalisé à des distances régulières au moyen d'un dispositif de transfert (11, 12), **caractérisé en ce que** lors du transfert, on retire d'abord la couche de support (9) des parties (4') contenant une ou plusieurs substances actives, puis les parties (3') de la couche auxiliaire en utilisant une couche de processus (5) réalisée de manière adhésive.

2. Procédé selon la revendication 1, **caractérisé en ce que** la bande (1) en forme de ruban et la deuxième bande (2) sont déplacées vers l'avant en cycle avec une phase de mouvement et de repos différente et/ou une longueur de pas différente et/ou une vitesse différente.

3. Procédé selon la revendication 1, **caractérisé en ce que** les dispositifs de transfert sont déplacés dans un mouvement de va-et-vient dans le sens de la bande lors du transfert des parties (4') contenant une ou plusieurs substances actives.
